# EUROPEAN PATENT APPLICATION

(11) **EP 4 751 765 A1**
(43) Date of publication of application: **03.06.2026**
(21) Application number: 24216153.7
(22) Date of filing: 28.11.2024
(51) Int. Cl.: A61N 1/37, A61B 5/363

(54) **IMPLANTABLE MEDICAL DEVICE HAVING AN OPTIMIZED UNDERSENSING MANAGEMENT**

(71) Applicant: BIOTRONIK SE & Co. KG, 12359 Berlin (DE)
(72) Inventor: Stuermer, Uwe, 10119 Berlin (DE); Wolf, Bernhard, 10707 Berlin (DE); Becker, Frank, 10409 Berlin (DE); Schneider, Peter, 12527 Berlin (DE); Noack, Christian, 12555 Berlin (DE)
(74) Representative: Biotronik Corporate Services SE

(57) **Abstract**

The present invention relates to an implantable medical device implantable in a human or animal heart that carries out the following steps during its operation: a) continuously detecting a cardiac electric signal (1); b) starting a coagulation timer (2) if the detected cardiac electric signal (1) is indicative for an atrial tachycardia; c) keeping the coagulation timer (2) active during a temporary loss (3) of the cardiac electric signal (1); and d) stopping the coagulation timer (2), if the following criteria are fulfilled: i) an amplitude of the cardiac electric signal (1) of a plurality of cardiac intervals being close in time exceeds a presettable significance threshold (8), and ii) an atrial rate calculated from the plurality of cardiac intervals being close in time is below a predefinable threshold rate indicative for atrial tachycardia, or e) keeping the coagulation timer (2) active for a predefined time period, if the criteria are not fulfilled.

## Description

The present invention relates to an implantable medical device, in particular an implantable medical device for stimulating a human or animal heart, according to the preamble of claim 1 and to a method for operating such an implantable medical device according to the preamble of claim 14.

Implantable medical devices for stimulating a human or animal heart, such as pacemakers, have been known for a long time. They can perform different functions. Different stimulation programs can be carried out by an appropriate pacemaker to restore the treated heart to a normal state.

A relevant feature of a pacemaker in connection with the detection of an atrial tachycardia (AT) or an atrial fibrillation (AF) is the capturing of the so-called coagulation time during such atrial tachycardic episode. The risk of an undesired blood coagulation due to an atrial tachycardia increases with the time the AT/AF state of the heart persists. Therefore, patients will typically not receive an antitachycardic therapy in a hospital if an atrial tachycardic episode persists for a longer time. In case of very weak atrial cardiac signals, an atrial tachycardic episode can be considered by an implantable medical device as terminated although it is physiologically still present. This technical termination or non-detection of an atrial tachycardia although it is still physiologically present is also referred to as atrial undersensing. A severe problem of atrial undersensing is an incorrect assessment of the coagulation time, i.e., of the risk of the patient to have a higher susceptibility of a mobilization of the coagulation due to an antitachycardic therapy during an ongoing atrial tachycardic episode.

It is an object of the present invention to provide an implantable medical device which increases the safety of the patient with regard to the risk of blood coagulation and mobilization thereof during antitachycardia therapy, in particular by having an improved atrial undersensing management during coagulation time tracking.

This object is achieved with an implantable medical device implantable in a human or animal heart, comprising a processor, a memory unit, and a detection unit configured to detect an electric signal of the same heart. The memory unit comprises a computer-readable program that causes the processor to perform the steps explained in the following when executed on the processor.

In one of the steps, the detection unit is used for continuously detecting a cardiac electric signal. This cardiac electric signal may comprise a plurality of peaks reflecting the cardiac activity of the patient.

In a further step, a coagulation timer is started if the detected cardiac electric signal is indicative for an atrial tachycardia (the term "atrial tachycardia" as used herein encompasses the term "atrial fibrillation"). This coagulation timer is understood to be a timer that counts a time during which the patient is assumed to be in a state of atrial tachycardia. The longer this state persists, the higher is the risk for the patient of a mobilization of the blood coagulation, even during (ineffective) antitachycardic therapy. Therefore, the timer is called "coagulation timer".

According to prior art techniques, the coagulation timer is stopped or even reset if cardiac signals indicative for a lower heart rate, in particular normal sinus rhythm, are detected by the detection unit. However, also poor sensing conditions (e.g. weak signals) can result in falsely detecting a termination of the atrial tachycardia and consequently in inappropriately stopping the coagulation timer.

In contrast to that, according to a further step performed by the presently claimed implantable medical device, the coagulation timer is kept active (kept counting the coagulation time) during a temporary loss of the cardiac electric signal. A temporary loss of the cardiac electric signal is understood to be a temporary non-detection of the cardiac electric signal, for instance due to temporary poor sensing conditions resulting in a cardiac signal too weak to be detected.

In a further step, the coagulation timer is stopped, if the following criteria are fulfilled: i) an amplitude of the cardiac electric signal of a plurality of cardiac intervals being close in time exceeds a presettable significance threshold, and ii) an atrial rate calculated from the plurality of cardiac intervals being close in time is below a predefinable threshold rate indicative for atrial tachycardia, or the coagulation timer is kept active for a predefined time period, if the criteria are not fulfilled.

The presently claimed implantable medical device enables an extended coagulation time tracking which is a relevant risk minimization measure. It enables that this coagulation time tracking is not prematurely stopped due to a purely technical reason like poor sensing conditions. Consequently, the presently claimed implantable medical device enables a better monitoring and more physiologic therapy of an atrial tachycardia than prior art devices do.

In an embodiment, the detection unit has a presettable sensing threshold, and the significance threshold is equal to or greater than 110% of the sensing threshold. In particular, the significance threshold may be equal to or greater than 115 %, in particular equal to or greater than 120 %, in particular equal to or greater than 125 %, in particular equal to or greater than 130 %, in particular equal to or greater than 140 %, in particular equal to or greater than 150 %, in particular equal to or greater than 160 %, in particular equal to or greater than 170 %, in particular equal to or greater than 180 %, in particular equal to or greater than 190 %, in particular equal to or greater than 200 %, of the previously set sensing threshold. If an amplitude of the cardiac electric signal exceeds the sensing threshold by this value, a reliable atrial sensing can be assumed which means that the detected significance threshold crossings likely constitute true atrial events. In other words, a heart rate evaluation based on such true atrial events is very likely to apply.

Furthermore, in an embodiment, the significance threshold may be dynamically adaptable according to the sensing threshold.

In another embodiment, the temporary loss of the cardiac electric signal is classified to be caused by a decrease in amplitude of the cardiac electric signal below the sensing threshold. A cardiac electric signal having an intensity below the sensing threshold is not detected by the detection unit. The reason for such an amplitude decrease may be a weakness of the cardiac electric signal or a temporal absence of any cardiac signal within a normal cardiac cycle. In the latter case, stopping the coagulation timer would not present any risk, if it would be an effect of normalization of the heart rate. In the first case, stopping the coagulation timer may be dangerous for the patient, since the atrial tachycardia might still persist, even if not detectable. However, by keeping the coagulation timer active in case of assuming the existence of such particularly weak signals, a very appropriate countermeasure against non-detection of an atrial signal (in the following also referred to as atrial undersensing) is taken. By assuming that the atrial rate is in fact higher than one would think when only evaluating sufficiently strong amplitudes in the detected cardiac signal, one can conclude that the previously observed atrial tachycardia is not yet terminated, but still persists. Therefore, the decision to keep the coagulation timer active is of high medical relevance.

In an embodiment, the sensing threshold is dynamically adjustable based on a peak amplitude of the cardiac electric signal. Thus, the sensing threshold is preferably a variable threshold that is increased after having detected an intense peak and that is then iteratively decreased. Such an automatic adaptation of the sensing threshold is often also referred to as "auto sense". During the method performed by the implantable medical device, the sensing threshold is preferably set to a value corresponding to a maximal sensitivity of the detection unit, i.e., to a lowest possible absolute value. If afterwards no intense peak is detected that can be safely assigned to a real cardiac activity, the sensing threshold will remain at this value for an extended period of time.

In an embodiment, the sensing threshold is equal to or smaller than a programmed maximal sensitivity of the detection unit.

In an embodiment, the sensing threshold lies in a range of from 0.2 mV to 6.7 mV, in particular from 0.3 mV to 6.6 mV, in particular from 0.4 mV to 6.5 mV, in particular from 0.5 mV to 6.0 mV, in particular from 0.6 mV to 5.5 mV, in particular from 0.7 mV to 5.0 mV, in particular from 0.8 mV to 4.5 mV, in particular from 0.9 mV to 4.0 mV, in particular from 1.0 mV to 3.5 mV, in particular from 1.5 mV to 3.0 mV, in particular from 2.0 mV to 2.5 mV. These values for the sensing threshold can be particularly well applied if the implantable medical device is an implantable cardioverter-defibrillator (ICD), a bipolar implantable pulse generator (IPG), or a unipolar IPG. In case of a (bipolar) ICD, a range of from 0.2 mV to 2.0 mV (including any of the sub-ranges indicated above) is particularly appropriate. In case of a unipolar IPG, a range of from 0.5 mV to 6.7 mV (including any of the above-mentioned sub-ranges) is particularly appropriate.

In an embodiment, a noise window is started when the amplitude of the cardiac electric signal exceeds the sensing threshold, wherein the noise window is a time period lying in a range of from 10 ms to 60 ms, in particular from 20 ms to 50 ms, in particular from 30 ms to 40 ms.

The noise window may be used to monitor a noise level of the detected cardiac signal. An increase in amplitude of the cardiac electric signal over the sensing threshold within a noise window is considered to be noise rather than a true signal. To safely distinguish such noise from true signals, the noise window is restarted every time a noise signal is detected within a noise window. A repeated restart of the noise window simply indicates that noise is present over a specific period of time and that the present amplitude increases are not true signals. A true signal caused by an increase in amplitude of the cardiac electric signal is only detected after the noise window has elapsed.

In an embodiment, the noise level is classified to exceed an acceptable noise threshold if the noise window has been restarted before elapse. In such a case, the noise window is prolonged. A repeated detection of noise within such a long noise window indicates that the measured cardiac signal is strongly interfered by noise and cannot be properly evaluated. This means that the heart rhythm, in particular presence or absence of a tachycardia, cannot be determined properly. Simultaneously, the repeated detection of noise indicates that there is no undersensing. In this case, a coagulation timer started upon earlier detected atrial tachycardia would continue to run.

In an embodiment, an undersensing monitoring window is started if the amplitude of the cardiac electric signal exceeds the sensing threshold and no previously started undersensing monitoring window is still running, or an undersensing monitoring window is restarted if the previously started undersensing monitoring window is still running and the amplitude of the cardiac electric signal exceeds the significance threshold. When starting or restarting an undersensing monitoring window, it is assumed that there is no undersensing at least for the time of the undersensing monitoring window.

In an embodiment, the undersensing monitoring window is a time period lying in a range of an expected atrial interval, in particular in a range of from 200 ms to 800 ms. Preferably, the undersensing monitoring window lies in a range of from 300 ms to 700 ms, in particular from 400 ms to 600 ms, in particular from 450 ms to 500 ms.

In an embodiment, the temporary loss of the cardiac electric signal, and thus a beginning of an undersensing phase, is detected when the undersensing monitoring window has elapsed. Furthermore, an end of the undersensing phase is preferably detected when the amplitude of the cardiac electric signal crosses the sensing threshold for the first time after the beginning of the undersensing phase.

In an embodiment, the coagulation timer is set to run for a time interval of from 12 hours to 72 hours, in particular of 24 hours or 36 hours or 48 hours, or in particular of from 20 hours to 50 hours, in particular of from 18 hours to 30 hours, in particular of from 24 hours to 28 hours. If the coagulation timer is active for such an extended period of time, the risk for the patient of experiencing an undesired mobilization of blood coagulation upon an antitachycardic therapy is significantly increased.

In an embodiment, the plurality of cardiac intervals being close in time (i.e., the plurality of cardiac intervals that is used to determine whether the coagulation timer is to be stopped or kept active) corresponds to a first natural number n of cardiac intervals for which the criteria are fulfilled within a second number m of consecutive cardiac intervals including cardiac intervals for which the criteria are fulfilled and cardiac intervals for which the criteria are not fulfilled, wherein n lies in a range of from 18 to 22 and m lies in a range of from 23 to 28, in particular n equals 20 and m equals 24. In another embodiment, the plurality of cardiac intervals corresponds to a plurality of consecutive cardiac intervals and lies in a range of from 2 to 40, in particular 10 to 40, in particular 4 to 30, in particular from 5 to 14, in particular from 6 to 13, in particular from 7 to 12, in particular from 8 to 11, in particular from 9 to 10.

Typically, an average or mean value is calculated from the number of consecutive cardiac intervals in order to determine the actual heart rate of the patient and to distinguish a normal or at least uncritical heart rate from a heart rate that can be assigned to atrial tachycardia.

In an embodiment, the predefinable threshold rate indicative for an atrial tachycardia lies in a range equal to or larger than 100 bpm, in particular in a range of from 100 bpm to 250 bpm corresponding to a range for a cardiac threshold interval of from 240 ms to 600 ms.

In an embodiment, the implantable medical device comprises a stimulation unit for stimulating the human or animal heart, wherein the computer-readable program causes the processor to prevent the stimulation unit from delivering an antitachycardic therapy if the coagulation timer has elapsed.

In an embodiment, the computer-readable program causes the processor to continue the coagulation timer from its latest value if the detected cardiac electric signal is, at a later time point, again indicative for an atrial tachycardia and if the coagulation timer has been kept active until this later time point. This ensures that the coagulation timer is not inadvertently reset, but rather continued from a first detection of the atrial tachycardic episode to a second detection of the presumed same atrial tachycardic episode, since it is highly likely that both detected atrial tachycardic episodes belong to one and the same physiologic atrial tachycardia that is only technically split up into two different episodes. By continuing the coagulation timer, the risk of developing a coagulation is much better reflected than in case of stopping and restarting (or even stopping, resetting and restarting) the coagulation timer. The risk for the patient of obtaining an antitachycardic therapy despite of the physiologic counter-indication is thus significantly reduced.

In an embodiment, after elapse of the undersensing monitoring window, the computer-readable program causes the processor to store information about a presence of an undersensing episode in the memory of the implantable device and/or to generate a signal indicating that an undersensing episode is present. Such a signal can be sent directly (without relevant temporal delay) to medical staff, e.g., via a home monitoring appliance. Additionally or alternatively, the signal can be provided to medical staff in the course of a regular reporting.

In an embodiment, the computer-readable program causes the processor to determine that noise is present if the noise level exceeds the acceptable noise threshold. Such a determination can be entered into an episode list entry (as part of the episode details), and can thus also be consulted to decide whether undersensing is present or not.

In an embodiment, the computer-readable program causes the processor to monitor an impedance of an electrode of the detection unit and to determine that an electrode error is present if the impedance lies outside a predefinable impedance range. Thus, this and the precedingly described embodiments enable an additional evaluation of noise and/or electrode impedance to decide whether undersensing is present.

In an embodiment, the computer-readable program causes the processor to assess a temporal change of observed amplitude peaks during an atrial tachycardic episode. For this purpose, all peak amplitudes of a detected atrial tachycardic episode and/or all values of the sensing threshold of the detection unit that are applied during a detected atrial tachycardic episode are stored in the memory unit of the implantable medical device. In addition, the temporal course of the peak amplitudes and/or the values of the sensing threshold are then evaluated. The obtained information on the temporal change of the peak amplitudes and/or the values of the sensing threshold can disclose additional information on the physiologic state of the patient and can help to decide on particularly appropriate therapies against any cardiac abnormalities that have been observed during operation of the implantable medical device.

An appropriate temporal course of the peak amplitudes and/or the values of the sensing threshold is the first derivative of the peak amplitudes and/or the values of the sensing threshold with time (d(peak amplitude)/dt and/or d(sensing threshold)/dt), i.e., the velocity by which the peak amplitudes and/or the values of the sensing threshold change (typically, they become smaller).

In an embodiment, the computer-readable program causes the processor to evaluate the detected cardiac electric signal and/or the peak amplitudes of the detected cardiac electric signal within the implantable medical device. Additionally or alternatively, the computer-readable program causes the processor to store the detected cardiac electric signal in the memory unit and to transfer the stored cardiac signal to an external evaluation device. If the cardiac electric signal and/or the peak amplitudes of the detected cardiac electric signal are evaluated within the implantable medical device, it is possible that the implantable medical device can immediately react in a way adapted to an evaluation result. Furthermore, such an evaluation of the cardiac electric signal and/or the peak amplitudes within the implantable medical device does not necessarily require storing the detected cardiac signal. This reduces the requirements for the memory unit of the implantable medical device. However, the calculation effort within the implantable medical device typically leads to an increased power consumption and might thus decrease the overall lifetime of the implantable medical device. If the detected cardiac electric signal is stored in the memory unit and transferred to an external evaluation device, it is not necessary to perform computationally intensive calculations within the implantable medical device. In addition, an evaluation of atrial tachycardic (AT) and/or atrial fibrillic (AF) episodes can be carried out over the whole set of interrelated or connected episodes. This may disclose additional medical information and can thus increase the knowledge of the physiologic state of the patient. Consequently, the chances to properly address the patient's physiologic needs can thus be increased in a highly appropriate way. Storing the detected cardiac signal in the memory unit, however, requires a sufficiently big dimensioned memory unit. In addition, the transfer of significantly more data per AT/AF episode results in a higher energy consumption and thus may also reduce the overall lifetime of the implantable medical device. Thus, it is an overall consideration between additional features and a possibly reduced lifetime that needs to be done in order to optimize the implantable medical device according to the specific needs for a certain group of patients or for certain applications.

In an aspect, the present invention relates to a method for operating an implantable medical device implantable in a human or animal heart, in particular an implantable medical device according to the preceding explanations. This method comprises the steps explained in the following.

In one of the steps, the detection unit is used for continuously detecting a cardiac electric signal. This cardiac electric signal usually comprises a plurality of peaks reflecting the cardiac activity of the patient.

In a further method step, a coagulation timer is started if the detected cardiac electric signal is indicative for an atrial tachycardia.

In a further method step, the coagulation timer is kept active during a temporary loss of the cardiac electric signal.

In a further method step, the coagulation timer is stopped if the following criteria are fulfilled: i) an amplitude of the cardiac electric signal of a plurality of cardiac intervals being close in time exceeds a presettable significance threshold, and ii) an atrial rate calculated from the plurality of cardiac intervals being close in time is below a predefinable threshold rate indicative for atrial tachycardia, or the coagulation timer is kept active for a predefined time period, if the criteria are not fulfilled.

In an aspect, the present invention relates to a method for treating an atrial tachycardia of a patient in need of such treatment, wherein this method comprises the steps explained in the following.

In one of the steps, a cardiac electric signal is continuously detected. This cardiac electric signal usually comprises a plurality of peaks reflecting the cardiac activity of the patient.

In a further method step, a coagulation timer is started if the detected cardiac electric signal is indicative for an atrial tachycardia.

In a further method step, the coagulation timer is kept active during a temporary loss of the cardiac electric signal.

In a further method step, the coagulation timer is stopped if the following criteria are fulfilled: i) an amplitude of the cardiac electric signal of a plurality of cardiac intervals being close in time exceeds a presettable significance threshold, and ii) an atrial rate calculated from the plurality of cardiac intervals being close in time is below a predefinable threshold rate indicative for atrial tachycardia, or the coagulation timer is kept active for a predefined time period, if the criteria are not fulfilled.

In a further method step, an antitachycardic pacing, by way of a stimulation unit of the implantable medical device, is applied to the patient only if the coagulation timer is active.

In an embodiment, detecting the atrial tachycardia is based on a plurality of detected atrial events in the cardiac electric signal, wherein an atrial event is detected when the amplitude of the cardiac electric signal exceeds the sensing threshold outside a noise window.

In an embodiment, an atrial tachycardia is detected when an atrial rate of a plurality of detected consecutive atrial events exceeds a tachycardic threshold rate. In another embodiment, an atrial tachycardia is detected when an atrial rate of a plurality of detected atrial events being close in time exceeds a tachycardic threshold rate. The plurality of cardiac intervals being close in time corresponds to a first natural number n of cardiac intervals for which the criteria are fulfilled within a second number m of consecutive cardiac intervals including cardiac intervals for which the criteria are fulfilled and cardiac intervals for which the criteria are not fulfilled, wherein n lies in a range of from 25 to 35 and m lies in a range of from 36 to 50, in particular n equals 36 and m equals 48. Whereas a termination of the atrial tachycardia may be detected when the atrial rate of a plurality of detected consecutive atrial events falls below the tachycardic threshold rate. In particular, a termination of the atrial tachycardia is detected when an atrial rate of at least two detected consecutive significant atrial events having an amplitude above the significance threshold falls below a tachycardic threshold rate or when an atrial rate of 36 cardiac intervals within a total number of 48 consecutive cardiac intervals having an amplitude above the significance threshold falls below a tachycardic threshold rate.

All embodiments of the implantable medical device can be combined in any desired way and can be transferred either individually or in any arbitrary combination to any of the described methods. Likewise, all embodiments of any of the methods can be combined in any desired way and can be transferred either individually or in any arbitrary combination to the implantable medical device or to the respective other method.

Further details of aspects of the present invention will be explained in the following making reference to exemplary embodiments and accompanying Figures. In the Figures:
- Figure 1: shows an implantable medical system comprising an implantable medical device according to an embodiment of the present invention;
- Figure 2: shows a first detail of an exemplary electrocardiogram illustrating an embodiment of the present invention;
- Figure 3: shows a second detail of the same electrocardiogram, wherein the second detail directly succeeds the first detail shown in Figure 2;
- Figure 4: shows a third detail of the same electrocardiogram, wherein the third detail directly succeeds the second detail shown in Figure 3;
- Figure 5: shows a fourth detail of the same electrocardiogram, wherein the fourth detail directly succeeds the third detail shown in Figure 4;
- Figure 6: shows a fifth detail of the same electrocardiogram, wherein the fifth detail directly succeeds the fourth detail shown in Figure 5; and
- Figure 7: shows a diagram comparing a method according to an embodiment of the present invention to a method of the state of the art.

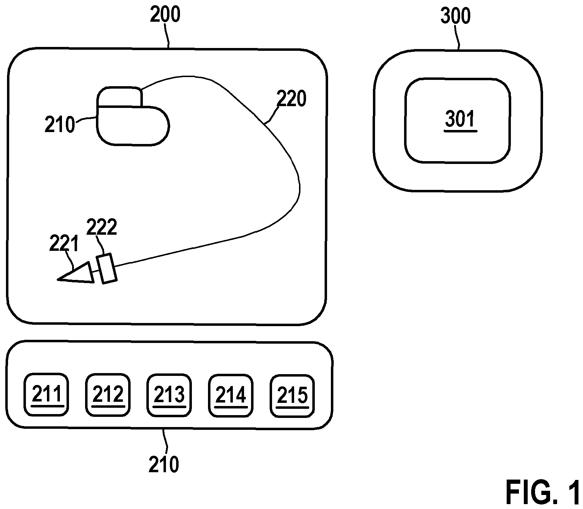

Figure 1 shows an implantable medical system 200 comprising an implantable medical device 210 according to an embodiment of the present invention. The implantable medical device 210 (in the following also denoted as generator device) is connectable to an electrode 220 having a distal tip electrode 221 and a ring electrode 222 arranged proximal from the tip electrode 221. The tip electrode 221 is configured to be anchored in cardiac tissue in the right atrium of a patient's heart. The generator device 210 comprises a processor 211, a detection unit 213, a memory unit 212, a stimulation unit 214 and a communication unit 215. The implantable medical device 210 is further configured to communicate with an external programming device 300 via communication unit 215. The external programming device 300 likewise comprises a communication unit 301 for communicating with the implantable medical device 210.

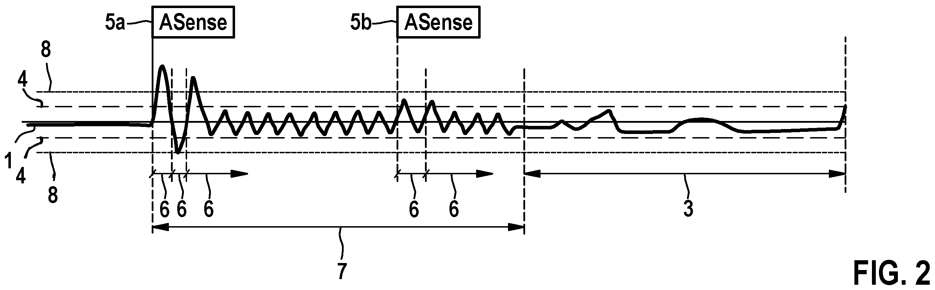

Figs. 2 to 6 show an operation of an algorithm which is run on the implantable generator device 210 to reliably detect a termination of an atrial tachycardia in the presence of occasionally weak cardiac electric signals and to meet the decision to stop or continue a running coagulation timer, in particular below an absolute sensing threshold 4 of the detection unit 213, which is known as atrial undersensing. Figs. 2 to 6 show an operation of the algorithm by means of an exemplary electrocardiogram which may be recorded by the detection unit 213 of the generator device 210 when implanted in a patient. Moreover, in Figs. 2 to 6 only so-called significant atrial events 5a are evaluated when detecting a termination of an atrial tachycardic episode, since particularly high safety requirements are imposed on the decision that a tachycardic phase has terminated and that a running coagulation timer can be stopped. A general determination of the atrial rhythm itself, in particular the detection of a presence of an atrial tachycardia, is carried out on the basis of all detected atrial events 5a and 5b.

Fig. 2 shows a first detail of an electrocardiogram in which the course of a cardiac electric signal 1 is presented. A sensing threshold 4 and a significance threshold 8 are set (both for positive and negative amplitude parts). In the following, the terms sensing threshold and significance threshold each mean absolute values of the sensing threshold and significance threshold, respectively. Upon the rise of the amplitude of cardiac electric signal 1 over the sensing threshold 4, a first atrial event 5a is detected. Simultaneously, a noise window 6 and an undersensing monitoring window 7 is started. The noise window 6 has a duration lying in a range of about 10 ms to 60 ms while the undersensing monitoring window 7 has a duration lying in a range of about 200 ms to 800 ms. Since in the further course, the amplitude of the cardiac electric signal 1 crosses the sensing threshold 4 again for two times within a noise window 6, the noise window 6 is restarted again for two times. Sense threshold crossings within a running noise window 6 are generally considered to be noise and no further events. Then, still within the running undersensing monitoring window 7, the noise window 6 ends and the signal 1 lies below the sensing threshold 4 such that no further atrial event is detected and no further noise window 6 is started. After some time, the amplitude of the cardiac electric signal 1 again rises over the sensing threshold 4 leading to a detection of a second atrial event 5b and simultaneously a noise window 6 is started. However, since at atrial event 5b the amplitude of the cardiac electric signal 1 does not exceed the significance threshold 8, the undersensing monitoring window 7 is not restarted, but continues to run. Within the running noise window 6, the amplitude of the cardiac electric signal 1 rises again over the sensing threshold 4 and is therefore classified to be noise rather than a further atrial event. After that, the amplitude of the cardiac electric signal 1 stays below the sensing threshold 4 until the undersensing monitoring window 7 elapses. Upon the elapse of the undersensing monitoring window 7, the cardiac electric signal 1 is considered to be lost, since the amplitude of the cardiac electric signal 1 does not exceed the significance threshold 8 and the undersensing monitoring window 7 is not restarted again (although an atrial event would be expected at that time, since the length of the undersensing monitoring window 7 corresponds to an expected normal cardiac interval), and an undersensing phase 3 starts. The signal 1 shows a normal heart rhythm having, however, a small amplitude below the sensing threshold 4 and is therefore not detectable by the detection unit 213. It is important to note again that the first atrial event 5a in Fig. 1 is a so-called significant atrial event, since, within an undersensing monitoring window 7, its amplitude not only crosses the sensing threshold 4, but also the significance threshold 8, while the amplitude of the second atrial event 5b only crosses the sensing threshold 4 which is therefore no significant atrial event and would therefore not be reliable enough to be used in the determination a termination of a probably existing atrial tachycardia leading to a deactivation of a probably active coagulation timer.

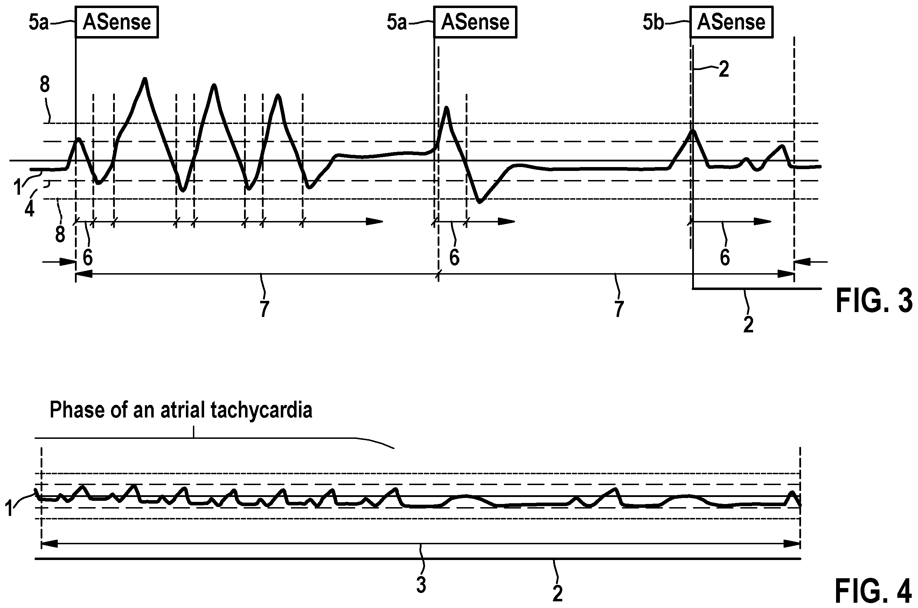

Fig. 3 shows a further detail of the electrocardiogram in which the course of a cardiac electric signal 1 is presented. At the end of the undersensing phase 3 of Fig. 2 the signal amplitude 1 rises again over the sensing threshold 4 producing a significant event 5a and starting an undersensing monitoring window 7 as well as a noise window 6. Even if the signal amplitude 1 does not rise over the significance threshold 8 here, this atrial event 5a is considered to be a significant, and thus reliable, event, since the detection occurred outside a running undersensing monitoring window 7. In the following, the noise window 6 is restarted again for seven times. All these amplitude crossings above the sensing threshold 4 causing the noise window restarts are considered to be noise and are not considered for signal evaluation. After that, the eighth noise window 6 elapses and the amplitude of the cardiac electric signal 1 remains below the sensing threshold 4 for some time, before it rises again over the significance threshold 8 and produces a significant atrial event 5a. Upon the rise over the significance threshold 8, the undersensing monitoring window 7 is restarted, since it has not been elapsed at that time. In addition, a new noise window 6 is started. The noise window 6 is restarted a second time, by the rise of the negative amplitude of the cardiac electric signal 1 before the amplitude remains below the sensing threshold 4 for some time. After that and still within the running undersensing monitoring window 7, the amplitude rises again over the sensing threshold 4, but does not reach the significance threshold 8, producing an atrial event 5b. At this atrial event 5b the noise window 6 is started, but the undersensing monitoring window 7 is not restarted. After the noise window 6 has elapsed, the cardiac electric signal 1 shows a further peak which, however, does not reach the sensing threshold 4. So, no noise window 6 is started and the undersensing monitoring window 7 elapses. The atrial event 5b is the beginning of a tachycardic episode. Details of the tachycardia detection algorithm are not described here. However, in general a plurality of events is necessary to fulfill certain detection criteria. Here, it is assumed that a presence of a tachycardia is detected at the atrial event 5b in Fig. 3. Upon the detection of the tachycardia, a coagulation timer 2 of 48 hours is started. During this time, an atrial antitachycardic pacing (aATP) therapy is applied by means of the implantable medical device 200 which is directed to a termination of the tachycardia. As soon as a termination of the tachycardia is detected, the coagulation timer 2 shall be stopped. If a termination is not detected and the coagulation timer 2 ends after 48 hours, the aATP therapy is stopped and a responsible physician is notified via the external programming device 300 or another external communications device.

Fig. 4 shows a further detail of the electrocardiogram presenting the further course of the cardiac electric signal 1. After the undersensing monitoring window 7 has elapsed at the end of Fig. 3, the cardiac electric signal 1 remains below the sensing threshold 4, which indicates a new undersensing phase 3. This undersensing phase 3 is thus detected by the detection unit 213 and is not confused with a wrongly detected termination of the tachycardic episode due to wrongly detecting an increase in the cardiac interval. As a result, the coagulation timer 2 is not stopped here, but continued to track the allowable time for aATP therapy. As shown in the first half of the electrocardiogram of Fig. 4, also during this undersensing phase 3, the cardiac electric signal 1 exhibits a tachycardic rhythm at least in the first half and continuing the coagulation timer 2 is indicated. Towards the end of the undersensing phase 3, the cardiac interval is increasing indicating that the atrial tachycardia is ending.

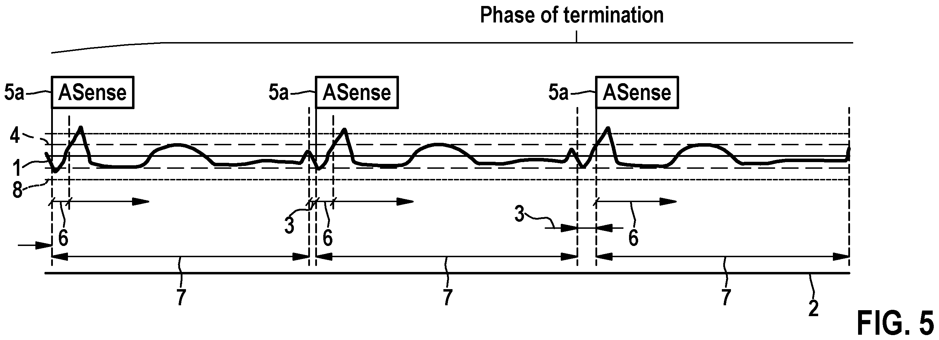

Fig. 5 shows a further detail of the electrocardiogram presenting the further course of the cardiac electric signal 1. The displayed cardiac electric signal 1 exhibits a termination phase of the tachycardia which means that the temporal distance of the atrial events is greater than a tachycardic interval that led to a detection of the tachycardia. The coagulation timer 2 is still running during the termination phase. The undersensing phase 3 of Fig. 4 ends by a crossing of the negative amplitude of the cardiac electric signal 1 over the sensing threshold 4 producing a significant atrial event 5a. Upon the crossing of the sensing threshold 4 an undersensing monitoring window 7 and a noise window 6 is started. The noise window 6 is restarted again by a further sensing threshold crossing of the positive amplitude of the cardiac electric signal 1. After the elapse of the undersensing monitoring window 7 a short undersensing phase 3 occurs which is terminated by a new significant atrial event 5a. Again an undersensing monitoring window 7 and a noise window 6 is started and again restarted. After elapse of the undersensing monitoring window 7, a short undersensing phase 3 follows again which is again terminated by a new significant atrial event 5a.

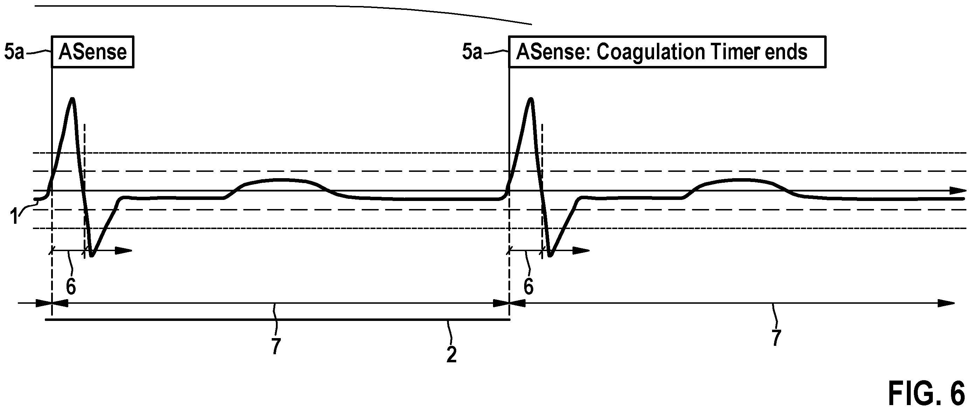

Fig. 6 shows a further detail of the electrocardiogram presenting the further course of the cardiac electric signal 1 which exhibits an end of the tachycardia. The two displayed significant atrial events lead to a detection of a termination of the tachycardia. In general, a plurality of atrial intervals are tested to finally detect a termination of the tachycardia. Upon detection of the termination of the tachycardia at the second atrial event 5a in Fig. 6 also the coagulation timer 2 is stopped. Both atrial events 5a again start an undersensing monitoring window 7 and a noise window 6 which is restarted once again before it elapses.

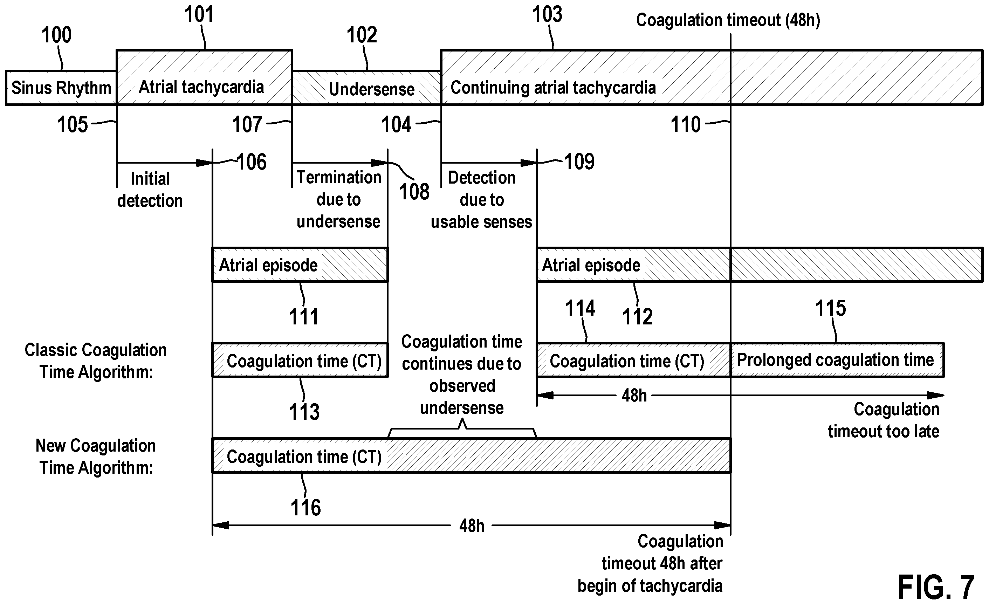

Fig. 7 shows a diagram comparing the coagulation tracking method according to an embodiment of the present invention to a method of the state of the art. Fig. 7 shows different phases 100-103 of a detected signal. The signal first exhibits a normal sinus rhythm 100. At a time point 105 the rhythm changes to a first tachycardic phase 101 which is detected after a detection phase at time point 106. After the first tachycardic phase 101 an undersensing phase 102 occurs at a time point 107. At time point 108, a technical termination of the atrial tachycardia due to undersensing is then detected at time point 108. At time point 104, the undersensing phase 102 has ended and a second phase 103 of the atrial tachycardia begins. The second tachycardic phase 103 is detected due to a detection of reliable atrial events at time point 109. Thus, without a particular undersensing monitoring, an implantable medical device is able to record a first atrial tachycardic episode 111 between the time points 106 and 108 and a second atrial tachycardic episode starting from the time point 109. According to prior art coagulation time tracking, an implantable medical device would therefore start a coagulation timer 113 at time point 106 upon detection of the presence of the tachycardia and would stop the coagulation timer 113 at time point 108 upon a false detection of a termination of the tachycardia due to undersensing. At time point 109 the coagulation timer 114 would be restarted upon detection of another tachycardia. Because of the restart of the coagulation timer 114 the atrial episode 112 at time point 109, the coagulation timer 114 elapses too late resulting in a prolonged coagulation time 115 which presents an increased risk for the patient of coagulation mobilization. In contrast to the prior art, an implantable medical device according to the present invention is able to detect the presence of the undersensing phase 102 and does not stop the coagulation timer 116 between the atrial episodes 111 and 112, since it assumes that the atrial tachycardia 101 persists during the undersensing phase 102 and continues afterwards in the second atrial tachycardic phase 103.

This prevents that the coagulation timer 116 elapses too late and the coagulation time is kept within a safe range.

## Claims

1. Implantable medical device (200) implantable in a human or animal heart, comprising a processor (211), a memory unit (212), and a detection unit (213) configured to detect an electric signal (1) of the same heart,
**characterized**
**in that** the memory unit (212) comprises a computer-readable program that causes the processor (211) to perform the following steps when executed on the processor (211):
a) continuously detecting, with the detection unit (213), a cardiac electric signal (1);
b) starting a coagulation timer (2) if the detected cardiac electric signal (1) is indicative for an atrial tachycardia;
c) keeping the coagulation timer (2) active during a temporary loss (3) of the cardiac electric signal (1); and
d) stopping the coagulation timer (2), if the following criteria are fulfilled:
i) an amplitude of the cardiac electric signal (1) of a plurality cardiac intervals being close in time exceeds a presettable significance threshold (8), and
ii) an atrial rate calculated from the plurality of cardiac intervals being close in time is below a predefinable threshold rate indicative for atrial tachycardia, or
e) keeping the coagulation timer active for a predefined time period, if the criteria are not fulfilled.

2. Implantable medical device according to claim 1, **characterized in that** the detection unit (213) has a presettable sensing threshold (4), and the significance threshold (8) is equal to or greater than 110% of the sensing threshold (4).

3. Implantable medical device according to claim 2, **characterized in that** the temporary loss (3) of the cardiac electric signal (1) is classified to be caused by a decrease of an amplitude of the cardiac electric signal (1) below the sensing threshold (4).

4. Implantable medical device according to claims 2 or 3, **characterized in that** the sensing threshold (4) is dynamically adjustable based on a peak amplitude of the cardiac electric signal (1).

5. Implantable medical device according to any one of claims 2 to 4, **characterized in that** the sensing threshold (4) is equal to or smaller than a programmed maximal sensitivity of the detection unit (213).

6. Implantable medical device according to any one of claims 2 to 5, **characterized in that** a noise window (6) is started when the amplitude of the cardiac electric signal (1) exceeds the sensing threshold (4), wherein the noise window (6) is a time period lying in a range of from 10 ms to 60 ms.

7. Implantable medical device according to any one of claims 2 to 6, **characterized in that** an undersensing monitoring window (7) is started if the amplitude of the cardiac electric signal (1) exceeds the sensing threshold (4) and no previously started undersensing monitoring window (7) is still running, or an undersensing monitoring window (7) is restarted if the previously started undersensing monitoring window (7) is still running and the amplitude of the cardiac electric signal (1) exceeds the significance threshold (8).

8. Implantable medical device according to any one of claims 2 to 7, **characterized in that** the undersensing monitoring window (7) is a time period lying in a range of an expected atrial interval, in particular in a range of from 200 ms to 800 ms.

9. Implantable medical device according to any one of claims 7 or 8, **characterized in that** the temporary loss (3) of the cardiac electric signal (1) is detected when the undersensing monitoring window (7) has elapsed.

10. Implantable medical device according to any one of the preceding claims, **characterized in that** the coagulation timer (2) is set to run for a time interval of from 12 hours to 72 hours, in particular of from 24 hours to 48 hours, in particular of 36 hours.

11. Implantable medical device according to any one of the preceding claims, **characterized in that** the plurality of cardiac intervals being close in time corresponds to a first natural number n of cardiac intervals for which the criteria are fulfilled within a second number m of consecutive cardiac intervals including cardiac intervals for which the criteria are fulfilled and cardiac intervals for which the criteria are not fulfilled, wherein n lies in a range of from 18 to 22 and m lies in a range of from 23 to 28, in particluar n equals 20 and m equals 24.

12. Implantable medical device according to any one of the preceding claims, **characterized in that** the predefinable threshold rate indicative for a tachycardia lies in a range equal to or larger than 100 bpm.

13. Implantable medical device according to any one of the preceding claims, **characterized in that** the implantable medical device comprises a stimulation unit (214) for stimulating the human or animal heart, wherein the computer-readable program causes the processor to prevent the stimulation unit from delivering an antitachycardic therapy if the coagulation timer has elapsed.

14. Method for operating an implantable medical device (210) implantable in a human or animal heart, the method comprising the following steps:
a) continuously detecting a cardiac electric signal (1);
b) starting a coagulation timer (2) if the detected cardiac electric signal (1) is indicative for an atrial tachycardia;
c) keeping the coagulation timer (2) active during a temporary loss (3) of the cardiac electric signal (1); and
d) stopping the coagulation timer (2), if the following criteria are fulfilled:
i) an amplitude of the cardiac electric signal (1) of a plurality of cardiac intervals being close in time exceeds a presettable significance threshold (8), and
ii) an atrial rate calculated from the plurality of cardiac intervals being close in time is below a predefinable threshold rate indicative for atrial tachycardia, or
e) keeping the coagulation timer active for a predefined time period, if the criteria are not fulfilled.

15. Method for treating an atrial tachycardia of a patient in need of such treatment, the method comprising the following steps:
a) continuously detecting a cardiac electric signal (1);
b) starting a coagulation timer (2) if the detected cardiac electric signal (1) is indicative for an atrial tachycardia;
c) keeping the coagulation timer (2) active during a temporary loss (3) of the cardiac electric signal (1);
d) stopping the coagulation timer (2), if the following criteria are fulfilled:
i) an amplitude of the cardiac electric signal (1) of a plurality of cardiac intervals being close in time exceeds a presettable significance threshold (8), and
ii) an atrial rate calculated from the plurality of cardiac intervals being close in time is below a predefinable threshold rate indicative for atrial tachycardia, or
e) keeping the coagulation timer (2) active for a predefined time period, if the criteria are not fulfilled; and
f) applying an antitachycardic pacing only if the coagulation timer (2) is active.
